(19) Europäisches Patentamt
European Patent Office
Office européen des brevets

(11) EP 3 290 417 A1

(12) **EUROPEAN PATENT APPLICATION**

(43) Date of publication:
**07.03.2018 Bulletin 2018/10**

(21) Application number: **16186658.7**

(22) Date of filing: **31.08.2016**

(51) Int Cl.:
*C07D 417/12 (2006.01)* *C07D 417/14 (2006.01)*
*A61K 31/506 (2006.01)* *A61P 1/00 (2006.01)*
*A61P 13/00 (2006.01)* *A61P 21/00 (2006.01)*
*A61P 25/28 (2006.01)* *A61P 27/02 (2006.01)*
*A61P 29/00 (2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **AXXAM S.p.A.**
**20091 Bresso (MI) (IT)**

(72) Inventors:
- **PEVARELLO, Paolo**
**20091 BRESSO (MI) (IT)**
- **SEVERI, Elda**
**20091 BRESSO (MI) (IT)**
- **SODANO, Mariangela**
**20091 BRESSO (MI) (IT)**
- **VITALONE, Rocco**
**20091 BRESSO (MI) (IT)**
- **PRANDI, Adolfo**
**20091 Bresso (MI) (IT)**

(74) Representative: **Bianchetti Bracco Minoja S.r.l.**
**Via Plinio, 63**
**20129 Milano (IT)**

(54) **2-CHLORO-N-[2-(1,3-THIAZOL-5-YL)ETHYL]-5-(5-FLUOROPYRIMIDIN-2-YL)-BENZAMIDES AND THEIR USE AS P2X7 RECEPTOR ANTAGONISTS**

(57) The present invention relates to novel substituted piperidine and morpholine compounds of formula (I)

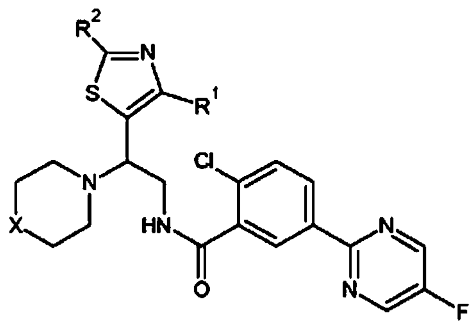

(I)

wherein
X is O or $CF_2$;
$R^1$ and $R^2$ are independently selected from the group consisting of hydrogen, $CH_2OH$, $CH_2F$, $CHF_2$, cyclopropyl, or -C≡CH with the proviso that when one of $R^1$ and $R^2$ is $CH_2OH$, $CH_2F$, $CHF_2$, cyclopropyl or -C≡CH, then the other of $R^1$ or $R^2$ is hydrogen, having P2X7 receptor (P2X7) antagonistic properties, pharmaceutical compositions comprising these compounds, chemical processes for preparing these compounds and their use in the treatment or prophylaxis of diseases associated with P2X7 receptor activity in animals, in particular humans.

EP 3 290 417 A1

## Description

The present invention is related to novel substituted piperidine and morpholine derivatives of formula (I) having P2X7 receptor (P2X7) antagonistic properties, pharmaceutical compositions comprising these compounds, chemical processes for preparing these compounds and their use in the treatment or prophylaxis of diseases associated with P2X7 receptor activity in animals, in particular humans.

P2X7 belongs to the family of P2X ionotropic receptors. P2X7 is activated by extracellular nucleotides, notably adenosine triphosphate (ATP). P2X7 is distinguished from other P2X family members by the specific localization (CNS and immunocompetent cells in particular), by the high concentrations of ATP (in the low mM range) required to activate it and by its ability to form a large pore upon prolonged or repeated stimulation. P2X7 is a ligand-gated ion channel and is present on a variety of cell types, largely those known to be involved in the inflammatory and /or immune process, specifically, macrophages, mast cells and lymphocytes (T and B). Activation of the P2X7 receptor by extracellular nucleotides, e.g., ATP, leads to the release of interleukin-1β (1L-1β) and giant cell formation (macrophages/ microglial cells), degranulation (mast cells) and L-selectin shedding (lymphocytes). P2X7 receptors are also located on antigen-presenting cells (APC), keratinocytes, salivary acinar cells (parotid cells), hepatocytes, erythrocytes, erythroleukaemic cells, monocytes, fibroblasts, bone marrow cells, neurones, and renal mesangial cells. The P2X7 receptor is also known to be a pain sensor in the nervous system. Experiments using P2X7 deficient mice demonstrate the role of P2X7 in the development of pain as these mice were protected from the development of both adjuvant-induced inflammatory pain and partial nerve ligation induced neuropathic pain. There is also growing evidence that P2X7 or its downstream effectors, such as IL-1β, are involved in the pathophysiology of several neurological disorders, such as, Alzheimer's Disease (J.I. Diaz-Hernandez et al., Neurobiol. Aging 2012, 1816-1828: In vivo P2X7 inhibition reduces Aβ plaques in AD through GSK3β). P2X7 is thought to have an important function in neurotransmission within the CNS through its activation on postsynaptic and/or presynaptic neurons and glia. Data has emerged using *in situ* hybridization that P2X7 receptor mRNA is widely distributed throughout the rat brain. Specifically, areas of high P2X7 mRNA expression were found in the anterior olfactory nucleus, cerebral cortex, piriform cortex (Pir), lateral septal nucleus (LS), hippocampal pyramidal cell layers of CA1, CA3, CA4, pontine nuclei, external cuneate nucleus, and medial vestibular nucleus. P2X7 hybridization signals were also observed in the motor neurons of the trigeminal motor nucleus, facial nucleus, hypoglossal nucleus, and the anterior horn of the spinal cord.

Hence there is a therapeutic rationale for the use of P2X7 antagonists in the treatment of a variety of disease states. These states include but are not limited to diseases associated with the CNS such as Alzheimer's Disease, Parkinson's Disease, Huntington's Disease, Anryotrophic Lateral Sclerosis, spinal cord injury, cerebral ischemia, head trauma, meningitis, sleep disorders, mood and anxiety disorders, HIV-induced neuroinflammation, and chronic neuropathic and inflammatory pain. Furthermore, peripheral inflammatory disorders and autoimmune diseases including but not limited to rheumatoid arthritis, ostheoarthritis, psoriasis, allergic dermatitis, asthma, chronic obstructive pulmonary disease, airways hyper-responsiveness, septic shock, bronchitis, glomerulonephritis, irritable bowel syndrome, fatty liver disease, liver fibrosis, skin injury, lung emphysema, muscular dystrophy, fibrosis, atherosclerosis, burn injury, Crohn's Disease, ulcerative colitis, age-related macular degeneration, growth and metastasis of malignant cells, Sjögren's syndrome, myoblastic leukaemia, diabetes, osteoporosis, ischemic heart disease are all examples where the involvement of P2X7 receptors has been implicated. In view of the clinical importance of P2X7, the identification of compounds that modulate P2X7 receptor function represents an attractive avenue into the development of new therapeutic agents.

P2X7 inhibitors are described in in various patent applications such as:

WO2004/099146 that discloses benzamide inhibitors of the P2X7 receptor and their use in the treatment of inflammatory diseases.

WO2009/108551 that discloses heteroarylamide analogs and their use in P2X7 receptor mediated conditions.

WO2009/132000 that discloses quinoline and isoquinoline substituted P2X7 receptor antagonists and their use in P2X7 receptor mediated conditions.

WO2015/118019 that discloses thiazole and oxazole derivatives as P2X7 receptor antagonists and their use in P2X7 receptor mediated conditions.

However there is still an unmet need for compounds which are able to efficiently antagonize P2X7 and that can be delivered in the different target organs which are sites of a P2X7 mediated pathology, including the brain. Such compounds are provided herein.

Various embodiments of the invention are presented hereafter;

The present invention relates to thiazole compounds of the following formula (I) or a pharmaceutically acceptable salt thereof:

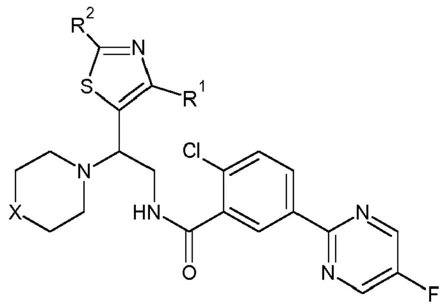

(I)

including any stereochemically isomeric form thereof, wherein

X is O or $CF_2$;
$R^1$ and $R^2$ are independently selected from the group consisting of hydrogen, $CH_2OH$, $CH_2F$, $CHF_2$, cyclopropyl, or -C=CH with the proviso that when one of $R^1$ and $R^2$ is $CH_2OH$, $CH_2F$, $CHF_2$, cyclopropyl or -C=CH, then the other of $R^1$ or $R^2$ group is hydrogen.

As used in the foregoing definitions:

The terms "halo", "halogen" and "halide", which may be used interchangeably, refer to a substituent fluoro, chloro, bromo, or iodo.

The term "stereochemically isomeric forms" as used hereinbefore defines all the possible isomeric forms which the compounds of formula (I) may possess. Unless other wise mentioned or indicated, the chemical designation of compounds denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the cis- or trans-configuration.

Stereochemically isomeric forms of the compounds of formula (I) are obviously intended to be embraced within the scope of this invention.

The absolute stereochemical configuration of the compounds of formula (I) and of the intermediates used in their preparation may easily be determined by those skilled in the art while using well-known methods such as, for example, X-ray diffraction.

Furthermore, some compounds of formula (I) and some of the intermediates used in their preparation may exhibit polymorphism. It is to be understood that the present invention encompasses any polymorphic forms possessing properties useful in the treatment of the conditions noted hereinabove.

The pharmaceutically acceptable salts as mentioned hereinabove are meant to comprise the therapeutically active non-toxic acid addition salt forms that the compounds of formula (I) are able to form. These pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methane sulfonic, trifluoromethanesulfonic, ethanesulfonic, benzene sulfonic, p-toluenesulfonic, cyclamic, salicylic, p-aminosalicylic, pamoic and the like acids.

Conversely said salt forms can be converted by treatment with an appropriate base into the free base form.

The compounds of formula (I) may exist in both unsolvated and solvated forms. The term 'solvate' is used herein to describe a molecular association comprising a compound of the invention and one or more pharmaceutically acceptable solvent molecules, e.g. water or ethanol. The term 'hydrate' is used when said solvent is water.

Most preferably, a compound of formula I according to this invention is selected from the group consisting of:

| Compound | IUPAC Name |
|---|---|
| 1 | (±)-2-chloro-N- {2-[2-(difluoromethyl)-1,3-thiazol-5-yl]-2-(morpholin-4-yl)ethyl}-5-(5-fluoropyrimidin-2-yl)benzamide |
| 2 | (+)-2-chloro-N- {2-[2-(difluoromethyl)-1,3-thiazol-5-yl]-2-(morpholin-4-yl)ethyl}-5-(5-fluoropyrimidin-2-yl)benzamide |

(continued)

| Compound | IUPAC Name |
|---|---|
| 3 | (-)-2-chloro-N- {2-[2-(difluoromethyl)-1,3-thiazol-5-yl]-2-(morpholin-4-yl)ethyl}-5-(5-fluoropyrimidin-2-yl)benzamide |
| 4 | 2-chloro-N- {2-[4-(cyclopropyl)-1,3-thiazol-5-yl]-2-(4,4-difluoropiperidin-1-yl)ethyl}-5-(5-fluoropyrimidin-2-yl)benzamide |
| 5 | 2-chloro-N- {2-[4-(hydroxymethyl)-1,3-thiazol-5-yl]-2-(4,4-difluoropiperidin-1-yl)ethyl}-5-(5-fluoropyrimidin-2-yl)benzamide |
| 6 | 2-chloro-N- {2-[4-(fluoromethyl)-1,3-thiazol-5-yl]-2-(4,4-difluoropiperidin-1-yl)ethyl}-5-(5-fluoropyrimidin-2-yl)benzamide |
| 7 | 2-chloro-N- {2-[2-(fluoromethyl)-1,3-thiazol-5-yl]-2-(morpholin-4-yl)ethyl}-5-(5-fluoropyrimidin-2-yl) benzamide |
| 8 | 2-chloro-N- {2-[2-(fluoromethyl)-1,3-thiazol-5-yl]-2-(4,4-difluoropiperidin-1-yl)ethyl}-5-(5-fluoropyrimidin-2-yl)benzamide |
| 9 | 2-chloro-N- {2-[2-(ethynyl)-1,3-thiazol-5-yl]-2-(morpholin-4-yl)ethyl}-5-(5-fluoropyrimidin-2-yl) benzamide |

Compounds of formula (I) can be synthesized in four different ways as described below.

In particular, compounds of formula (I) are obtained by reacting a compound of formula (II):

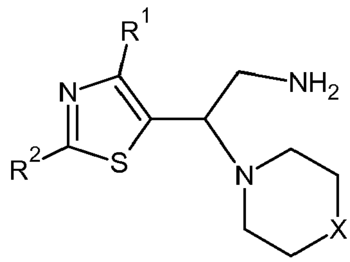

(II)

wherein the meanings of X, $R^1$, and $R^2$ are as defined above, with a compound of formula (III)

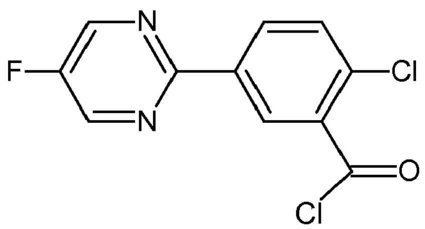

(III)

The reaction of a compound of formula (II) with a compound of formula (III), may be carried out in a at least one reaction-inert solvent and in the presence of a suitable base thereof at room temperature for 30 minutes.

Compound of formula (III) is known in the art or can be prepared following the processes reported in the examples.

Compounds of formula (II) can be prepared according to the following scheme:

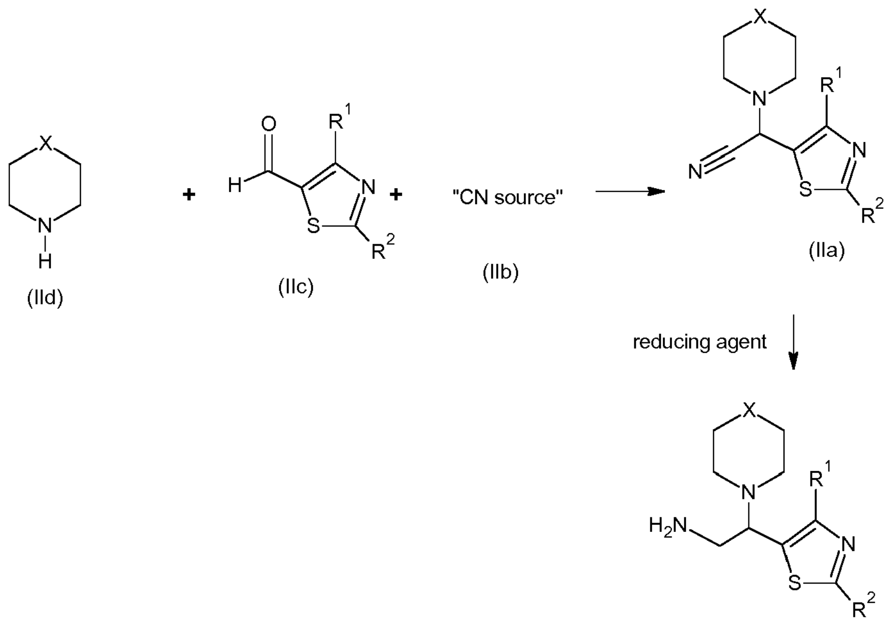

(IId) (IIc) (IIb) (IIa)

Primary amines (II) can be obtained by reduction of the respective nitrile derivatives (IIa) in a nitrogen-hydrogen bond forming reaction. Non-limiting examples of such reaction include reduction with:

- hydrogen or a hydrogen source in the presence of a metal such as nickel, platinum, palladium and cobalt or a derivative thereof such as Ni-Raney, platinum oxide, palladium oxide or Raney cobalt as catalyst;
- a hydride such as lithium aluminum hydride, diisobutylaluminum hydride (DIBAL), boron hydride or a functional derivative thereof.

The reaction may be performed in a suitable solvent, such as methanol, tetrahydrofuran, acetic acid, diethyl ether, toluene or methanolic ammonia solution preferably at temperatures between -78°C and RT.

Compounds of formula (IIa), wherein $R^1$, and $R^2$ are as defined in formula (I), can be prepared from commercially available aldehydes (IIc) by a Strecker condensation reaction with the respective heterocyclyl intermediate (IId) in presence of a source of cyanide (IIb) for example TMSCN or a functional derivative thereof, in a solvent such as AcOH or MeCN, preferably at temperatures between 0°C and RT.

Stirring may enhance the rate of the Strecker condensation reaction. The starting materials and some of the intermediates are known compounds and are commercially available or may be prepared according to conventional reaction procedures generally known in the art.

The said process further optionally comprising asymmetric reaction using chiral auxiliaries based synthesis (using carbohydrate, chiral amine or cyclic ketimine) and/or catalytic asymmetric Strecker synthesis (using guanidine, chiral Schiff base or BINOL-based catalyst).

Compounds of formula (I) are also obtained by reacting a compound of formula (IV)

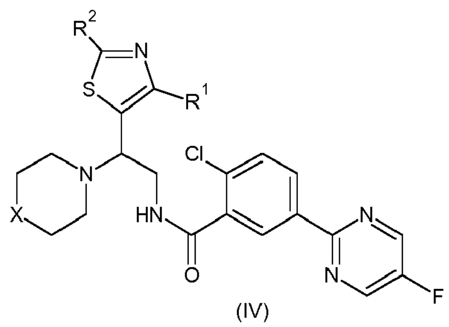
(IV)

wherein the meanings of X, $R^1$ and $R^2$ are as defined above, with commercially available fluorinating agent.

This reaction may be performed in a reaction-inert solvent such as, for example DCM, and in the presence of deoxofluor solution. Temperature can range between 0°C and the room temperature of the reaction mixture for 35 minutes.

When $R^2$ is CHO group, compounds of formula (IV) can be prepared from the oxidation of appropriate compound (Iva):

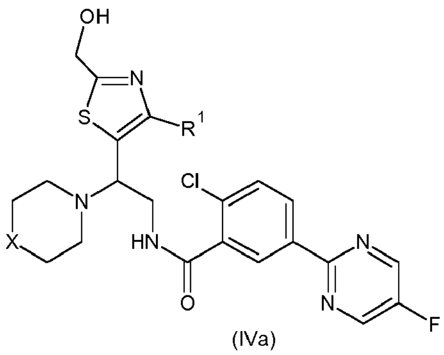

(IVa)

obtained from the coupling reaction between the opportune amine (II) with the chloride (II)I.

Primary amines (II) can be obtained as described above starting from not commercially available intermediate 1a synthesized according to the following scheme:

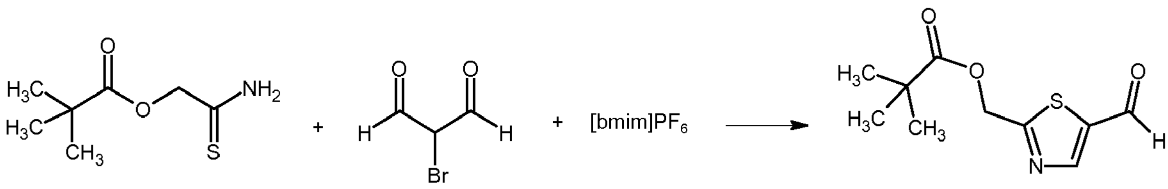


1a

(1a) is known in the art or can be prepared following the processes reported in the examples.

Compounds of formula (I) are also obtained from the hydrolysis of opportune compound of formula (V)

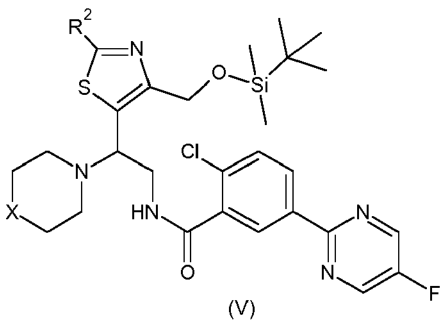

(V)

wherein the meanings of X and $R^2$ are as defined above, using acetic acid anf commercially available TBAF in THF for 30 minutes at room temperature.

The compounds of formula (V) can be obtained from the coupling reaction between the opportune amine (II) with the chloride (II)I.

Primary amines (II) can be obtained as described above starting from not commercially available intermediate (If) synthesized according to the following scheme:

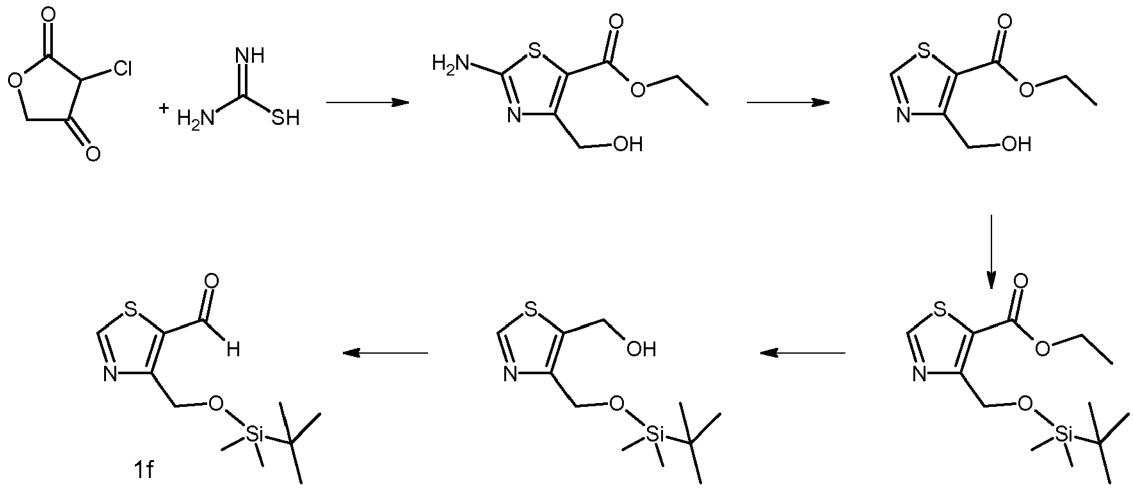

1f

Compound (If) can be prepared following the processes reported in the examples.

Finally, compounds of formula (I) were prepared from the opportune compound of formula (VI)

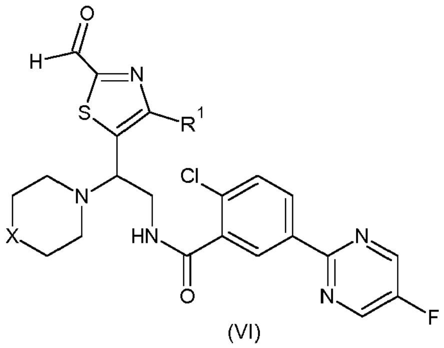

(VI)

wherein the meanings of X and $R^1$ are as defined above, by ethynylation.

This reaction may be performed for example at room temperature, in dry alchol, preferably MeOH, using for example the commercially available Ohira-Bestmann reagent. The time required to accomplish the reaction yield is 30 minutes-2 hours, preferably 1 hour.

Compounds of formula (VI) can be obtained from the corresponding intermediates (IVa) (which are synthesized as described above) by oxidation reaction.

The compounds of formula (I) as prepared in the hereinabove described processes may be synthesized in the form of racemic mixtures of enantiomers which can be separated from one another following art-known resolution procedures. Those compounds of formula (I) that are obtained in racemic form may be converted into the corresponding diastereomeric salt forms by reaction with a suitable chiral acid. Said diastereomeric salt forms are subsequently separated, for example, by selective or fractional crystallization and the enantiomers are liberated there from by alkali. An alternative manner of separating the enantiomeric forms of the compounds of formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The compounds of formula (I), the pharmaceutically acceptable salts and stereoisomeric forms thereof possess P2X7 receptor antagonizing properties as demonstrated in the Pharmacological Examples. Other examples of art-known group transformation reactions to convert compounds of formula (I) into other compounds of formula (I) are hydrolysis of carboxylic esters to the corresponding carboxylic acid or alcohol; hydrolysis of amides to the corresponding carboxylic acids or amines; alcohols may be converted into esters and ethers; primary amines may be converted into secondary or tertiary amines; double bonds may be hydrogenated to the corresponding single bond. The starting materials and some of the intermediates are known compounds and are commercially available or may be prepared according to conventional reaction procedures generally known in the art. The compounds of formula (I) as prepared in the hereinabove described processes may be synthesized in the form of racemic mixtures of enantiomers which can be separated from

one another following art-known resolution procedures. Those compounds of formula (I) that are obtained in racemic form may be converted into the corresponding diastereomeric salt forms by reaction with a suitable chiral acid. Said diastereomeric salt forms are subsequently separated, for example,by selective or fractional crystallization and the enantiomers are liberated there from by alkali. An alternative manner of separating the enantiomeric forms of the compounds of formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials. In the preparation of the compounds of formula I and the starting materials and/or intermediates described herein it may be useful to protect certain groups which are sensitive to the reaction conditions. The evaluation of the usefulness of the optional protection, as well as the selection of the suitable protecting agent, according to the reaction carried out in the preparation of the compounds of the invention and the functional group to be protected, are within the common knowledge of the skilled person. The removal of the optional protective groups is carried out according to conventional techniques. For a general reference to the use of protective groups in orgamic chemistry, see Theodora W. Greene and Peter G.M. Wuts "Protective groups in organic synthesis", John Wiley & Sons, Inc., II Ed., 1991.

The preparation of the salts of the compounds of formula (I) is carried out according to known methods. Therefore the present compounds of formula (I) are useful as a medicine especially in the treatment of a condition or disease mediated by the P2X7 receptor, in particular P2X7 receptor antagonistic activity. Subsequently the present compounds may be used for the manufacture of a medicine for treatment of a condition or a disease mediated by P2X7 receptor activity, in particular P2X7 receptor antagonistic activity.

The present invention also provides the use of a compound of formula (I) or a pharmaceutically acceptable salt thereof for the manufacture of a medicament for the treatment of conditions or diseases selected from P2X7 receptor mediated conditions or diseases. In an embodiment, the present invention provides a compound of formula (I) for use as a medicine or for use in the treatment of conditions or diseases selected from P2X7 receptor mediated conditions or diseases. Further, the present invention also provides a method of treatment of a condition mediated by P2X7 receptor activity, in a mammalian subject, which method comprises administering to a mammal in need of such treatment a therapeutically effective amount of a compound of formula (I) or a pharmaceutically acceptable salt thereof. In view of the above described mechanisms of action, the compounds of the invention are useful for the treatment of neurodegenerative disorders of various origins such as Alzheimer's Disease and other dementia conditions such as Lewys body, fronto-temporal dementia and taupathies; amyotrophic lateral sclerosis, Multiple Sclerosis, Parkinson's Disease and other parkinsonian syndromes; HIV-induced neuroinflammation; essential tremors; other spino cerebellar degenerations and Charcot-Marie-Toot neuropathy. The compounds of the invention are also useful for the treatment of neurological conditions such as epilepsy including simple partial seizure, complex partial seizure, secondary generalized seizure, further including absence seizure, myoclonic seizure, clonic seizure, tonic seizure, tonic clonic seizure and atonic seizure.

The compounds of the invention are also useful for the treatment of cognitive disorders and of psychiatric disorders. Psychiatric disorders include, and are not limited to major depression, dysthymia, mania, bipolar disorder (such as bipolar disorder type I, bipolar disorder type II), cyclothymic disorder, rapid cycling, ultradian cycling, mania, hypomania, schizophrenia, schizophreniform disorders, schizoaffective disorders, personality disorders, attention disorders with or without hyperactive behaviour, delusional disorders, brief psychotic disorders, shared psychotic disorders, psychotic disorder due to a general medical condition, substance-induced psychotic disorders or a psychotic disorder not otherwise specified, anxiety disorders such as generalised anxiety disorder, panic disorders, post-traumatic stress disorder, impulse control disorders, phobic disorders, dissociative states and moreover in smoke, drug addiction and alcoholism. In particular bipolar disorders, psychosis, anxiety and addiction.

The compounds of the present invention are useful in the prevention or treatment of neuropathic pain. Neuropathic pain syndromes include, and are not limited to: diabetic neuropathy; sciatica; non-specific lower back pain; multiple sclerosis pain; fibromyalgia; HIV-related neuropathy; neuralgia, such as post-herpetic neuralgia and trigeminal neuralgia, Morton's neuralgia, causalgia; and pain resulting from physical trauma, amputation, phantom limb, cancer, toxins or chronic inflammatory conditions; central pain such as the one observed in thalamic syndromes, mixed central and peripheral forms of pain such as complex regional pain syndromes (CRPS) also called reflex sympathetic dystrophies.

The compounds of the invention are also useful for the treatment of chronic pain. Chronic pain includes, and is not limited to, chronic pain caused by inflammation or an inflammatory-related condition, ostheoarthritis, rheumatoid arthritis, acute injury or trauma, upper back pain or lower back pain (resulting from systematic, regional or primary spine disease such as radiculopathy), bone pain (due to osteoarthritis, osteoporosis, bone metastasis or unknown reasons), pelvic pain, spinal cord injury-associated pain, cardiac chest pain, non-cardiac chest pain, central post-stroke pain, myofascial pain, sickle cell pain, cancer pain, Fabry's disease, AIDS pain, geriatric pain or pain caused by headache, temporomandibular joint syndrome, gout, fibrosis or thoracic outlet syndromes, in particular rheumatoid arthritis and osteoarthritis.

The compounds of the invention are also useful in the treatment of acute pain caused by acute injury, illness, sport-medicine injuries, carpal tunnel syndrome, burns, musculoskeletal sprains and strains, musculotendinous strain, cervicobrachial pain syndromes, dyspepsis, gastric ulcer, duodenal ulcer, dysmenorrhea, endometriosis or surgery (such as open heart or bypass surgery), post operative pain, kidney stone pain, gallbladder pain, gallstone pain, obstetric pain or dental pain.

The compounds of the invention are also useful in the treatment of headaches such as migraine, tension type headache, transformed migraine or evolutive headache, cluster headache, as well as secondary headache disorders, such as the ones derived from infections, metabolic disorders or other systemic illnesses and other acute headaches, paroxysmal hemicrania and the like, resulting from a worsening of the above mentioned primary and secondary headaches.

Compounds of the invention are also useful in the treatment of diseases such as vertigo, tinnitus, muscle spasm, and other disorders including and not limited to cardiovascular diseases (such as cardiac arrhythmia, cardiac infarction or angina pectoris, hypertension, cardiac ischemia, cerebral ischemia) endocrine disorders (such as acromegaly or diabetes insipidus) diseases in which the pathophysiology of the disorder involves excessive or hypersecretory or otherwise inappropriate cellular secretion of an endogenous substance (such as catecholamine, a hormone or a growth factor).

The compounds of the invention are also useful in the selective treatment of liver disease, such as inflammatory liver diseases, for example chronic viral hepatitis B, chronic viral hepatitis C, alcoholic liver injury, primary biliary cirrhosis, autoimmune hepatitis, liver fibrosis, non-alcoholic steatohepatitis and liver transplant rejection.

The compounds of the invention inhibit inflammatory processes affecting all body systems. Therefore are useful in the treatment of inflammatory processes of the muscular-skeletal system of which the following is a list of examples but it is not comprehensive of all target disorders: arthritic conditions such as alkylosing spondylitis, cervical arthritis, fibromyalgia, gout, juvenile rheumatoid arthritis, lumbosacral arthritis, osteoarthritis, osteoporosis, psoriatic arthritis, rheumatic disease; disorders affecting skin and related tissues: eczema, psoriasis, dermatitis and inflammatory conditions such as sunburn; disorders of the respiratory system: asthma, allergic rhinitis and respiratory distress syndrome, lung disorders in which inflammation is involved such as asthma and bronchitis; chronic obstructive pulmonary disease; disorders of the immune and endocrinological systems: periarthritis nodosa, thyroiditis, aplastic anaemia, scleroderma, myasthenia gravis, multiple sclerosis and other demyelinizating disorders, encephalomyelitis, sarcoidosis, nephritic syndrome, Bechet’s syndrome, polymyositis, gingivitis.

Compounds of the invention are also useful in the treatment of gastrointestinal (GI) tract disorders such as inflammatory bowel disorders including but not limited to ulcerative colitis, Crohn’s disease, ileitis, proctitis, celiac disease, enteropathies, microscopic or collagenous colitis, eosinophilic gastroenteritis, or pouchitis resulting after proctocolectomy and post ileonatal anastomosis, and irritable bowel syndrome including any disorders associated with abdominal pain and/or abdominal discomfort such as pylorospasm, nervous indigestion, spastic colon, spastic colitis, spastic bowel, intestinal neurosis, functional colitis, mucous colitis, laxative colitis and functional dyspepsia; but also for treatment of atrophic gastritis, gastritis varialoforme, ulcerative colitis, peptic ulceration, pyrosis, and other damage to the GI tract, for example, by Helicobacter pylori, gastroesophageal reflux disease, gastroparesis, such as diabetic gastroparesis; and other functional bowel disorders, such as non-ulcerative dyspepsia (NUD); emesis, diarrhoea, and visceral inflammation.

Compounds of the invention are also useful in the treatment of disorders of the genito-urinary tract such as overactive bladder, prostatitis (chronic bacterial and chronic non-bacterial prostatitis), prostadynia, interstitial cystitis, urinary incontinence and benign prostatic hyperplasia, annexities, pelvic inflammation, bartholinities and vaginitis. In particular, overactive bladder and urinary incontinence.

The compounds of the invention are also useful in the treatment of ophthalmic diseases such as retinitis, retinopathies, uveitis and acute injury to the eye tissue, age-related macular degeneration or glaucoma, conjunctivitis.

The compounds of the invention are also useful in the treatment of eating disorders such as anorexia nervosa including the subtypes restricting type and binge-eating/purging type; bulimia nervosa including the subtypes purging type and non-purging type; obesity; compulsive eating disorders; binge eating disorder; and eating disorder not otherwise specified.

The compounds of the invention are also useful in the treatment of allergie dermatitis, hyperresponsiveness of the airway ,chronic obstructive pulmonary disease (COPD), bronchitis, septic shock, Sjögren’s syndrome, glomerulonephritis, atherosclerosis, growth and metastases of malignant cells, myoblastic leukaemia, diabetes, meningitis, osteoporosis, burn injury, ischaemic heart disease, stroke, peripheral vascular disease, varicose veins, glaucoma.

The term "treating" and "treatment’, as used herein, refers to curative, palliative and prophylactic treatment, including reversing, alleviating, inhibiting the progress of, or preventing the disease, disorder or condition to which such term applies, or one or more symptoms of such disease, disorder or condition.

Additionally the present invention provides pharmaceutical compositions comprising at least one pharmaceutically acceptable carrier and a therapeutically effective amount of a compound of formula (I).

In order to prepare the pharmaceutical compositions of this invention, an effective amount of the particular compound, in base or acid addition salt form, as the active ingredient is combined in intimate admixture with at least one pharmaceutically acceptable carrier, which carrier may take a wide variety of forms depending on the form of preparation desired for administration. These pharmaceutical compositions are desirably in unitary dosage form suitable, preferably, for oral administration, rectal administration, percutaneous administration or parenteral injection.

For example in preparing the compositions in oral dosage form, any of the usual liquid pharmaceutical carriers may be employed, such as for instance water, glycols, oils, alcohols and the like in the case of oralliquid preparations such as suspensions, syrups, elixirs and solutions; or solid pharmaceutical carriers such as starches, sugars, kaolin, lubricants, binders, disintegrating agents and the like in the case of powders, pills, capsules and tablets. Because of their easy administration, tablets and capsules represent the most advantageous oral dosage unit form, in which case solid pharmaceutical carriers are obviously employed. For parenteral injection compositions, the pharmaceutical carrier will mainly comprise sterile water, although other ingredients may be included in order to improve solubility of the active ingredient.

Injectable solutions may be prepared for instance by using a pharmaceutical carrier comprising a saline solution, a glucose solution or a mixture of both. Injectable suspensions may also be prepared by using appropriate liquid carriers, suspending agents and the like. In compositions suitable for percutaneous administration, the pharmaceutical carrier may optionally comprise a penetration enhancing agent and/or a suitable wetting agent, optionally combined with minor proportions of suitable additives which do not cause a significant deleterious effect to the skin. Said additives may be selected in order to facilitate administration of the active ingredient to the skin and/or be helpful for preparing the desired compositions. These topical compositions may be administered in various ways, e.g., as a transdermal patch, a spot-on or an ointment. Addition salts of the compounds of formula (1), due to their increased water solubility over the corresponding base form, are obviously more suitable in the preparation of aqueous compositions.

It is especially advantageous to formulate the pharmaceutical compositions of the invention in dosage unit form for ease of administration and uniformity of dosage.

"Dosage unit form" as used herein refers to physically discrete units suitable as unitary dosages, each unit containing a predetermined amount of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

For oral administration, the pharmaceutical compositions of the present invention may take the form of solid dose forms, for example, tablets (both swallowable and chewable forms), capsules or gelcaps, prepared by conventional means with pharmaceutically acceptable excipients and carriers such as binding agents (e.g. pregelatinised maize starch, polyvinylpyrrolidone, hydroxypropylmethylcellulose and the like), fillers (e.g. lactose, microcrystalline cellulose, calcium phosphate and the like), lubricants (e.g. magnesium stearate, tale, silica and the like), disintegrating agents (e.g. potato starch, sodium starch glycollate and the like), wetting agents (e.g. sodium laurylsulphate) and the like. Such tablets may also be coated by methods well known in the art.

Liquid preparations for oral administration may take the form of e.g. solutions, syrups or suspensions, or they may be formulated as a dry product for admixture with water and/or another suitable liquid carrier before use. Such liquid preparations may be prepared by conventional means, optionally with other pharmaceutically acceptable additives such as suspending agents (e.g. sorbitol syrup, methylcellulose, hydroxypropylmethylcellulose or hydrogenated edible fats), emulsifying agents (e.g. lecithin or acacia), non-aqueous carriers (e.g. almond oil, oily esters or ethyl alcohol), sweeteners, flavours, masking agents and preservatives (e.g. methyl or propyl p-hydroxybenzoates or sorbic acid).

Pharmaceutically acceptable sweeteners useful in the pharmaceutical compositions of the invention comprise preferably at least one intense sweetener such as aspartame, acesulfame potassium, sodium cyclamate, alitarne, a dihydrochalcone sweetener, monellin, stevioside sucralose (4,1',6'-trichloro-4, 1',6'-trideoxygalactosucrose) or, preferably, saccharin, sodium or calcium saccharin, and optionally at least one bulk sweetener such as sorbitol, mannitol, fructose, sucrose, maltose, isomalt, glucose, hydrogenated glucose syrup, xylitol, caramel or honey. Intense sweeteners are conveniently used in low concentrations. For example, in the case of sodium saccharin, the said concentration may range from about 0.04% to 0.1% (weight/volume) of the final formulation. The bulk sweetener can effectively be used in larger concentrations ranging from about 10% to about 35%, preferably from about 10% to 15% (weight/volume). The pharmaceutically acceptable flavours which can mask the bitter tasting ingredients in the low-dosage formulations comprise preferably fruit flavours such as cherry, raspberry, black currant or strawberry flavour. A combination of two flavours may yield very good results. In the high-dosage formulations, stronger pharmaceutically acceptable flavours may be required such as Caramel Chocolate, Mint Cool, Fantasy and the like.

Each flavour may be present in the final composition in a concentration ranging from about 0.05% to 1% (weight/volume). Combinations of said strong flavours are advantageously used. Preferably a flavour is used that does not undergo any change or loss of taste and/or color under the circumstances of the formulation.

The compounds of formula (I) may be formulated for parenteral administration by injection, conveniently intravenous, intra-muscular or subcutaneous injection, for example by bolus injection or continuous intravenous infusion. Formulations for injection may be presented in unit dosage form, e.g. in ampoules or multi-dose containers, including an added preservative. They may take such forms as suspensions, solutions or emulsions in oily or aqueous vehicles, and may contain formulating agents such as isotonizing, suspending, stabilizing and/or dispersing agents. Alternatively, the active ingredient may be present in powder form for mixing with a suitable vehicle, e.g. sterile pyrogen-free water, before use.

The compounds of formula (I) may also be formulated in rectal compositions such as suppositories or retention enemas, e.g. containing conventional suppository bases such as cocoa butter and/or other glycerides.

Those of skill in the treatment of diseases linked to the mediation of the ligand-gated ion channels will easily determine the therapeutically effective amount of a compound of formula (I) from the test results presented hereinafter. In general it is contemplated that a therapeutically effective dose will be from about 0.001 mg/kg to about 50 mg/kg of body weight, more preferably from about 0.01 mg/kg to about 10 mg/kg of body weight of the patient to be treated. It may be appropriate to administer the therapeutically effective dose in the form of two or more sub-doses at appropriate intervals throughout the day. Said sub-doses may be formulated as unit dosage forms, for example each containing from about 0.1 mg to about 1000 mg, more particularly from about 1 to about 500 mg, of the active ingredient per unit dosage form.

As used herein, a "therapeutically effective amount" of a compound, is the quantity of a compound which, when administered to an individual or animal, results in a sufficiently high level of that compound in the individual or animal to cause a discernible P2X7 receptor antagonistic response.

The exact dosage and frequency of administration depends on the particular compound of formula (I) used, the particular condition being treated, the severity of the condition being treated, the age, weight and general physical condition of the particular patient as well as the other medication, the patient may be taking, as is well known to those skilled in the art. Furthermore, said "therapeutically effective amount" may be lowered or increased depending on the response of the treated patient and/or depending on the evaluation of the physician prescribing the compounds of the instant invention. Theeffective daily amount ranges mentioned hereinabove are therefore only guidelines.

**Nomenclature and Structures**

In general, the nomenclature used in this Application is based on ChemSketch™ (ACDLabs) and generated according to the IUPAC systematic nomenclature. Chemical structures shown herein were prepared using ISIS® version 2.2. Any open valency appearing on a carbon, oxygen, sulfur, or nitrogen atom in the structures herein indicates the presence of a hydrogen atom unless indicated otherwise. Where a nitrogen-containing heteroaryl ring is shown with an open valency on a nitrogen atom and variables such as $R^1$, $R^2$, $R^3$ etc. are shown on the heteroaryl ring, such variables may be bound or joined to the open valency nitrogen. Where a chiral center exists in a structure but no specific stereochemistry is shown for the chiral center, both enentiomers associated with the chiral center are encompassed by the structure. Where a structure shown herein may exist in multiple tautomeric forms, all such tautomers are encompassed by the structure. The atoms represented in the structure herein are intended to encompass all naturally occurring isotopes of such atoms. Thus, for example, the hydrogen atoms represented herein are meant to include deuterium and tritium, and the carbon atoms are meant to include $^{13}C$ and $^{14}C$ isotopes.

**Abbreviations**

Abbreviations which may be used in the description of the Schemes and the Examples that follows are:

AcOH: Acetic acid
Anh: Anhydrous
AcONa: Sodium acetate
Boc: Tert-butyl-carbonate
$Boc_2O$: Di-tert-butyl dicarbonate
CC: Column Chromatography
DAST: Diethylaminosulfur trifluoride
DCM: Dichloromethane
DEA: Diethylamine
DIAD: Diisopropylazodicarboxylate
DIBAL: Diisobutylaluminiumhydride
DIPEA: Diisopropylethylenamine
DMAP: Dimethylaminopyridine
DMF: Dimethylformamide

DMSO: Dimethylsulfoxide
$Et_2O$: Diethyl ether
EtOAc: Ethyl acetate
EtOH: Ethanol
ESI: Electrospray ionization
HBTU: N,N,N',N'-Tetramethyl-O-(1H-benzotriazol-1-yl)uronium hexafluorophosphate;
h: hour;
Hrs hours
M: Molar
MeCN: Acetonitrile
MeOH: Methanol
Min: Minute(s)
Ni-Raney : Nickel-Raney
NMR: Nuclear Magnetic Resonance
rt: Room Temperature
TFA Trifluoroacetic acid
THF: Tetrahydrofurane;
TLC: Thin Layer Chromatography
TMSCN Trimethylsilylcyanide;
UPLC-MS: UltraPerformance LiquidChromatography-Mass Spectrometry
XPhos: 4,5-bis(diphenylphosphino)-9,9-dimethylxantene.

**Experimental part**

**The** following examples illustrate the present invention. Unless explicitly stated otherwise, all particulars (especially percentages and amounts) relate to the weight.

**Synthesis of the intermediates 1a-1e**

a) Preparation of

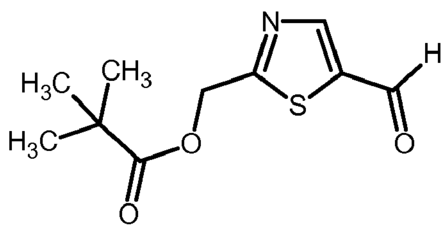

intermediate **1a**

To a suspension of 2-bromomalonaldehyde (17.1 mmol, 1 eq.) in 1.5 mL of the ionic liquid [bmim]$PF_6$, was added 2-amino-2-thioxoethyl 2,2-dimethylpropanoate (17.1 mmol, 1 eq.). Then, the reaction mixture was diluted with DCM (6 mL), sonicated for 5 minutes and magnetically stirred at room temperature overnight. An yellowish precipitate was obtained and from TLC analysis (EP/EtOAc 4:1 v/v), starting was consumed. The solvent was evaporated under reduced pressure and the product was purified by flash chromatography on $SiO_2$ using EP/EtOAc 4:1 v/v as the eluant to afford the intermediate **1a** as a viscous yellow oil (Y: 46%).

a) Preparation of

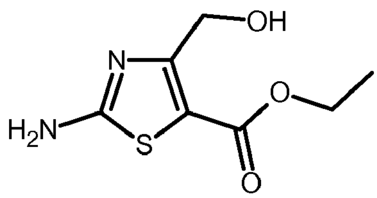

intermediate **1b**

A solution of 3-chlorofuran-2,4(3H,5H)-dione (74.3 mmol, 1eq.) and thiourea (86.2 mmol, 1.2 eq.) in EtOH (50 mL) was heated at reflux for 4 hrs. Solvent was removed and the residue was dissolved in water with HCl (1M). The aqueous solution was basified with aqueous $Na_2CO_3$; the solid formed was filtered, washed with water and dried in vacuo (Y: 73%).

b) Preparation of

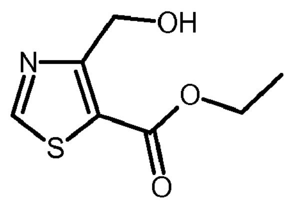

intermediate **1ac**

Intermediate **1b** (11 mmol, 1eq.) was dissolved in EtOH (75 mL) and AcOH (67 mL); $NaNO_2$ (237 mmol, 20eq.) and $NaHSO_3$ (237 mmol, 20eq.) were added and the reaction mixture was stirred at rt for 2 hrs. $H_2O$ (100 mL) and DCM (50 mL) were added to the reaction mixture. The aqueous phase was extracted with DCM (3x 50 mL) and was washed with saturated $NaHCO_3$ (1x100 mL). The combined organic phases were dried (anh. $Na_2SO_4$), evaporated and dried in vacuo to remove all AcOH and give intermediate **1c** as an orange/red oil (Y: 85%).

c) Preparation of

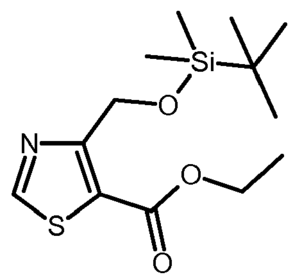

intermediate **1d**

Intermediate **1c** (10.7 mmol, 1eq.) was dissolved in DCM (10 mL); imidazole (21.4 mmol, 2eq.) was added one-pot and the reaction solution became orange. A solution of TBDMS-Cl (12.8 mmol, 1.2 eq.) in DCM (10 mL) was added at 0°C and the reaction mixture was stirred overnight at rt. Then, after LC-MS analysis, DMF (3 mL) was added and after 10 minutes, TBDMS-Cl (6.4 mmol, 0.6 eq.) and imidazole (13.9 mmol, 1.3 eq.) were added. The reaction mixture was stirred for 90 min at rt and then putted in fridge for all the week-end. DCM was removed under vacuum, the residue was diluted with $Et_2O$ (100 mL) and washed with water. The organic phase was dried (anh. $Na_2SO_4$), filtered and evaporated. The crude was purified by FC over $SiO_2$ (SNAP 50g: EP/EtOAc 8:2 v/v) to afford intermediate **1d** as a yellow oil (Y: 84%).

c) Preparation of

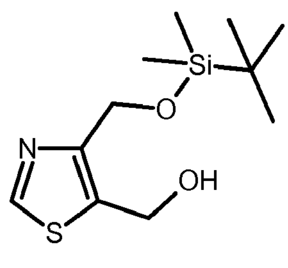

intermediate **1e**

Intermediate **1d** (8.96 mmol, 1eq.) was dissolved in DCM dry (20 mL). The mixture was cooled to -78°C and DIBAL in DCM (1M) (9.85 mmol, 1.1 eq.) was added dropwise. After 2 h, the mixture was quenched adding $H_2O$ (0.5 mL) and Rochelle salt (15 mL) and was stirred overnight. The organic phase was collected, dried (anh. $Na_2SO_4$) and finally evaporated under reduced pressure. The crude was purified by FC over $SiO_2$ (SNAP 100g: EP/EtOAc 8:2 v/v) to

give intermediate **1e** (Y: 19.4%)

c) Preparation of

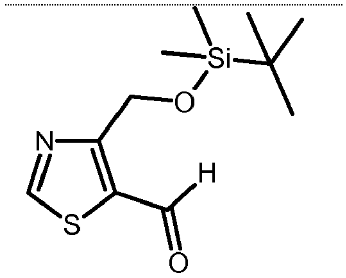

intermediate **1f**

Intermediate **1e** (1.73 mmol, 1eq.) was dissolved in DCM (4 mL); $MnO_2$ (25.3 mmol, 14.6 eq.) was added and the reaction mixture was stirred overnight at rt. Then, after LC-MS analysis, $MnO_2$ (7.87 mmol, 4.5eq.) was added. After 3 hrs the reaction was stopped. The mixture was diluted with EtOAc and was filtered over a pad of celite, which was washed several times with EtOAc. The combined organic phases were then concentrated to give intermediate **1f** used in the next step without any purification.

**Preparation of $\alpha$-aminonitriles 2a-2d**

**General procedure**

Aldehyde (3.30 mmol, 1eq.), opportune amine (6.60 mmol, 2 eq.) and AcONa (16.5 mmol, 5 eq.) were magnetically stirred in AcOH (15 mL) for 3 hrs. Then, the reaction mixture was cooled to 0°C and TMSCN (13.2 mmol, 4 eq.) was added dropwise and the mixture was allowed to warm to rt and stirred overnight. Then, was cooled to 0°C and was quenched with $H_2O$ (attention of HCN emission). After warming up to room temoperature, the mixture was stirred for 30 min in open vessel and the solvent was evaporated under reduced pressure. The resulting solid was taken up with DCM, water was added and was basified with $K_2CO_3$ until disappearance of effervescence (pH aqueous layer = 8). The organic layer was separated, dried (anh. $Na_2SO_4$), filtered and evaporated under reduced pressure. The crude was purified by FC ($SiO_2$) with EP/EtOAc 4:1 v/v as the eluant giving the desired $\alpha$-aminonitrile (Y: 41-87%).

Using this procedure,

Intermediate **2a** (Y: 61%) was prepared starting from intermediate **1a** and morpholine (commercially available), purchased from chemical providers.

Intermediate **2b** (Y: 87%) was prepared starting from intermediate **1a** and 4,4-difluoropiperidine (commercially available), purchased from chemical providers.

Intermediate **2c** (Y: 72%) was prepared starting from 4-cyclopropyl-l,3-thiazole-5-carbaldehyde (commercially available) and morpholine (commercially available), purchased from chemical providers.

Intermediate **2d** (Y: 41%) was prepared starting from intermediate **If** and 4,4-difluoropiperidine hydrochloride (commercially available), purchased from chemical providers.

| **2a** | **2b** | **2c** | **2d** |
|---|---|---|---|
| 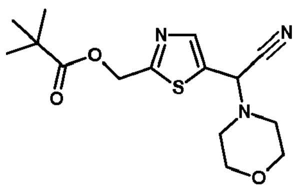 | 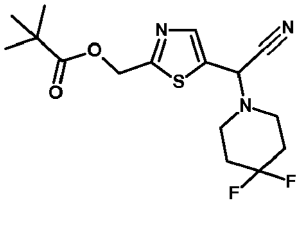 | 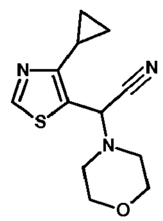 | 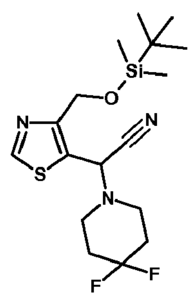 |

**Preparation of diamines 3a-3d**

**Procedure n°1**

ALPHA-aminonitrile (0.77 mmol, 1 eq.) was dissolved in $Et_2O$ (10 mL); the reaction mixture was cooled to 0°C and $LiAlH_4$ 1M in THF(2.31 mmol; 3 eq.) was added dropwise. After 25 min, from the LC-MS analysis, there was no trace of starting, so the mixture was quenched adding $Et_2O/H_2O$ (25 mL/1.5 mL); after stirring for 15 min at rt, EtOAc (75 mL) was added and the mixture was stirred again for 15 min, dried over $Na_2SO_4$, filtered and finally evaporated under reduced pressure. The crude product was purified by CC on $SiO_2$ gel using EtOAc / MeOH / $NH_3$ 9:1:1 v/v/v as the eluent to give the desired diamines (Y: 24-95%).

Using this procedure,

Intermediate **3a** (Y: 24% after purification) was prepared starting from intermediate **2a.**

Intermediate **3b** (Y: 95% without purification) was prepared starting from intermediate **2b.**

**Procedure n°2**

ALPHA-aminonitrile (0.43 mmol, 1 eq.) was dissolved in MeOH (10 mL) and was added $NH_3$ in MeOH 7M (1.15 mL). The solution was hydrogenates with H-Cube using Ni-Raney cartridge at 35°-60°C, 40-60 atm and 1mL/min. When the starting was consumed (UPLC-MS) the reaction was stopped; then, the Ni-Raney catridge was washed with MeOH/$NH_3$ and the solvent was evaporated under reduced pressure. During the hydrogenation was accumulated water inside reaction, so the mixture was extracted with DCM and concentrated; the crude product was used without purification (Y: 44-67%).

Using this procedure,

Intermediate **3c** (Y: 44%) was prepared starting from intermediate **2c.**

Intermediate **3d** (Y: 67%) was prepared starting from intermediate **2d.**

| **3a**<br>**AXX013_0600_010** | **3b**<br>**A0025_31_01** | **3c**<br>**A0022_33_01** | **3d**<br>**A0022_63_01** |
|---|---|---|---|
| 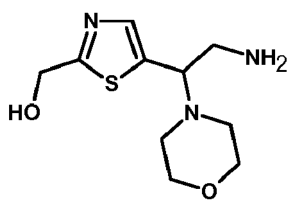 | 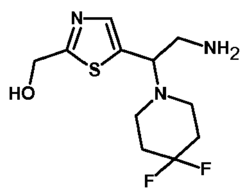 | 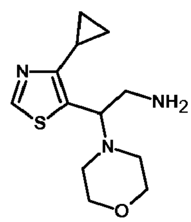 | 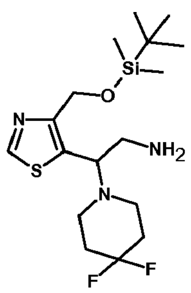 |

**Preparation of intermediates 4a and 4b**

a) Preparation of

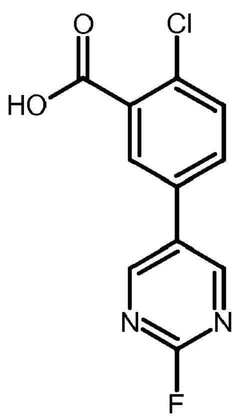

intermediate **4a**

Into a two-neck 50mL round-bottom flask, equipped with a mechanical stirred and reflux condenser, was dissolved 5-borono-2-chlorobenzoic acid (5.0 mmol, 1 eq.) and $Cs_2CO_3$ (7.5 mmol, 1.5 eq.) in degassed mixture of DMF/$H_2O$ (8.0/4.0 mL). To the resulting solution were added 2-chloro-5-fluoropyrimidine (6.25 mmol, 1.25 eq.) and $Pd(PPh_3)_4$ (0.1 mmol, 0.02 eq.) and the reaction mixture was heated at 110°C for 24 hrs. Then, the solvent was evaporated under reduced pressure. The resulting solid was dissolved in $H_2O$, basified with NaOH 3M until pH=12, and extracted with $Et_2O$ (1x 20mL) and DCM (1x 20mL). The aqueous phase was acidified by 1M HCl until pH=1, the precipitate was filtered, washed with water and dried under vacuum, to obtained intermediate **4a** as a pale yellow solid. (Y: 94%)

a) Preparation of

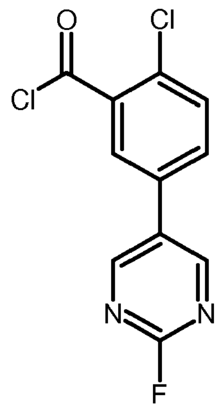

intermediate **4b**

Intermediate 4a (21.57 mmol, 1eq.) was dissolved in $SOCl_2$ (120 mL) and was heated to reflux (90°C) for 4 hrs. The solvent was removed under reduced pressure, and the crude was stripped several times with DCM to afford intermediate **4b** as a white solid, used in the next synthetic step without further purification (Y: 99%).

**Preparation of intermediates 5a, 5b and 5c**

**General procedure**

To a solution of opportune diamine intermediate **3** (1.03 mmol; 1eq.) in DCM (12 mL) was added a suspension of intermediate **4b** (2.57 mmol; 2.5 eq.) in DCM (8 mL). After 10 min, TEA (3.60 mmol; 3.5 eq.) was added and the reaction mixture was stirred for 15 min (LC-MS analysis). The solvent was removed under vacuum; the crude was dissolved in THF (12 mL) and a solution of NaOH 25M (12 mL) was added. After 30 min (LC-MS analysis), HCl 1M was added dropwise up to pH=7. THF was removed under vacuum and the residue was diluted with DCM (50 mL). Organic phase was washed with $NaHCO_3$ (2x100 mL) and $H_2O$ (100 mL); then was dried over $Na_2SO_4$, filtered and finally evaporated under reduced pressure. The crude was purified by CC on $SiO_2$ using DCM/MeOH 98:2 v/v to 95:5 v/v as the eluant to afford the desired product (Y: 57-67%).

Using this procedure,
Intermediate **5a** (Y: 57%) was prepared starting from intermediate 3a.

Intermediate **5b** (Y: 99%, without purification) was prepared starting from intermediate 3b.

| **5a** | **5b** |
|---|---|
| 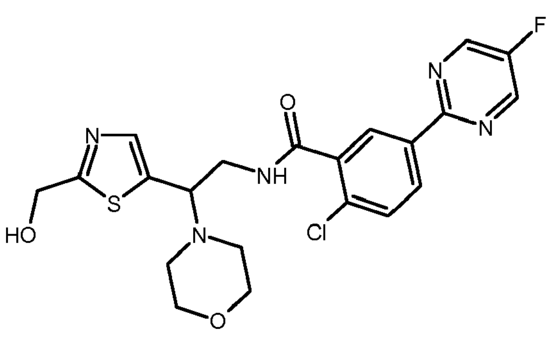 | 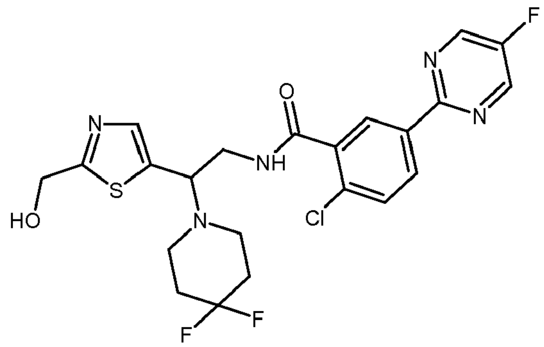 |

a) Preparation of

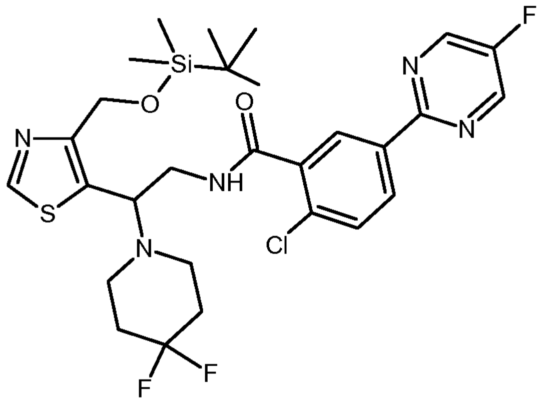

intermediate **5c**

Intermediate **3d** (0.29 mmol, 1eq.) was dissolved in DCM (2 mL) and added to a stirred solution of interemediate **4b** (0.29 mmol, 1eq.) in DCM (3 mL). The reaction mixture was stirred for 35 min; from the UPLC-MS, there was a bit trace of starting and there was many acid so TEA (not dry) (40 μL, 1eq.) was added. After 20 min, the reaction was finished (UPLC-MS analysis). The reaction mixture was sequentially washed with 5% aqueous citric acid (2x 10mL) and sat. $NaHCO_3$ (2x 10 mL). The combined organic phases were dried (anh. $Na_2SO_4$) and evaporated. The crude was purified by FC over $SiO_2$ (SNAP 25g: EP/EtOAc 7:3 to 6:4 v/v) to give intermediate **5c** as a yellow oil (Y: 60%)

**Preparation of intermediates 6a and 6b**

**General procedure**

Opportune intermediate **5** (0.125 mmol; 1 eq.) was dissolved in DCM (0.85 mL); $MnO_2$ (1.75 mmol; 14 eq.) was added and the reaction mixture was stirred overnight at rt. The mixture was then filtered over a pad of celite, which was washed several times with DCM. The resulting organic phase was concentrated under reduced pressure and the crude product was purified by CC on $SiO_2$ using DCM/MeOH 98:2 v/v to 95:5 v/v as the eluant to afford the desired intermediate **6** (Y: 51-80%).

Using this procedure,

Intermediate **6a** (Y: 51 %) was prepared starting from intermediate **5a.**

Intermediate **6b** (Y: 80%) was prepared starting from intermediate **3b.**

| 6a | 6b |
|---|---|
| 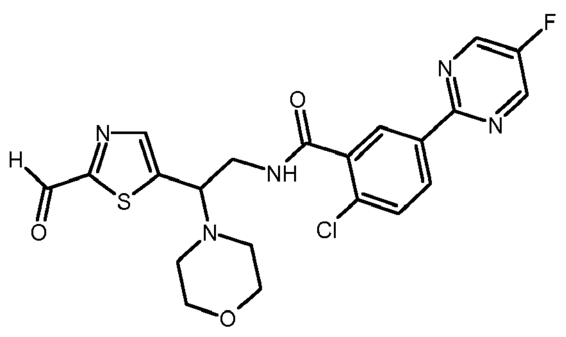 | 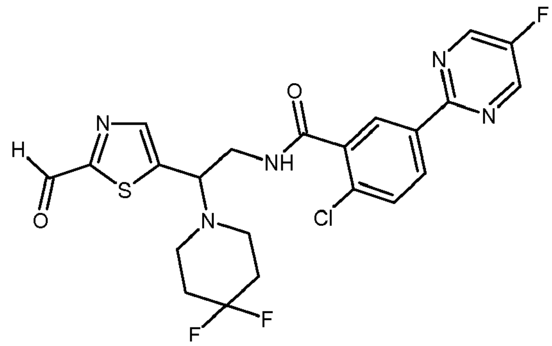 |

General procedure for the synthesis of final compounds

**Preparation of Compound 4**

Intermediate **3c** (0.079 mmol, 1eq.) was dissolved in DCM (1.5 mL); TEA dry (0.158 mmol, 2eq.) and intermediate **4b** (0.095 mmol, 1.2 eq.) were sequentially added at rt. After 30 min from the UPLC-MS the reaction was finished.

The reaction mixture was sequentially washed with 5% aqueous citric acid (10 mL) and sat. $NaHCO_3$ (10 mL).The combined organic phases were dried (anh. $Na_2SO_4$) and evaporated.

The crude was purified by HPLC giving Example 4 (Y: 64%).

**Preparation of Compound 5**

Intermediate **5c** (0.17 mmol, 1eq.) was dissolved in THF (2.7 mL); AcOH (5 drops) and TBAF (1M) (1.25 mmol, 7 eq.) in THF (1.2 mL) were added at rt. After 30 min from the UPLC-MS the reaction was finished.

The reaction mixture was basified with sat. $NaHCO_3$ until the end of effervescence and was washed with $H_2O$ (2x 15 mL). The combined organic phases were dried (anh. $Na_2SO_4$) and evaporated. The crude was purified by FC over $SiO_2$ (SNAP 10g: EP/EtOAc 2:8 to 9:1 v/v) to afford Example **5** as a colorless oil (Y: 86%).

**Preparation of Compound 9**

To a mixture of intermediate **6b** (0.057 mmol, 1.0 eq.) and $K_2CO_3$ (0.114 mmol, 2.0 eq.) in dry MeOH (0.7 mL), which was left stirring for 30 minutes at rt, Ohira-Bestmann reagent (0.068 mmol, 1.2 eq.) was added and the reaction mixture was stirred at rt for 30 min.The resulting solution was diluited with $Et_2O$ and washed with sat. $NaHCO_3$ (3x 10 mL), the organic phases were collected, dried (anh. $Na_2SO_4$) and evaporated under reduced pressure. The crude was purified by FC over $SiO_2$ (SNAP 10g: EP/EtOAc 8:2 to 6:4 v/v) to give Example **9** (Y: 26%).

**Preparation of Compounds 1-6-7-8**

**General procedure**

The opportune intermediate, alcohol intermediate **5** or aldehyde intermediate **6** precursor, (0.042 mmol; 1 eq.) was dissolved in DCM (1.6 mL); deoxo-fluor solution (50% in toluene) (0.1 mL) was added at 0°C and the reaction mixture was stirred at rt for 35 min (LC-MS analysis). Then, the reaction mixture was diluted with DCM and washed with $NaHCO_3$ (2x10 mL) and $H_2O$ (10 mL). After drying over $Na_2SO_4$ and filtration, the solvent was evaporated under reduced pressure to give the crude product. This was purified by HPLC to afford the pure desired product (Y: 24-51 %).

Using this procedure compounds:

**Compound 1** (Y: 51 %) was prepared starting from intermediate **6a.**
**Compound 2** was separated from Compound 1 as described under system purification
**Compound 3** was separated from Compound 1 as described under system purification
**Compound 6** (Y: 24%) was prepared starting from Example **5.**
**Compound 7** (Y: 24%) was prepared starting from intermediate **5a.**
**Compound 8** (Y: 24%) was prepared starting from was prepared starting from intermediate **5b.**

**Table 1** lists final compounds that were prepared and tested according to the experimental procedure described above. /

| Compound | Structure | IUPAC Name |
|---|---|---|
| 1 | 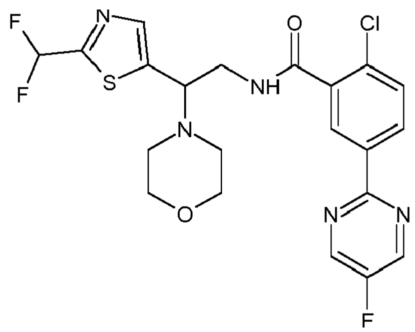 | (±)-2-chloro-N-{2-[2-(difluoromethyl)-1,3-thiazol-5-yl]-2-(morpholin-4-yl)ethyl}-5-(5-fluoropyrimidin-2-yl)benzamide |
| 2 | 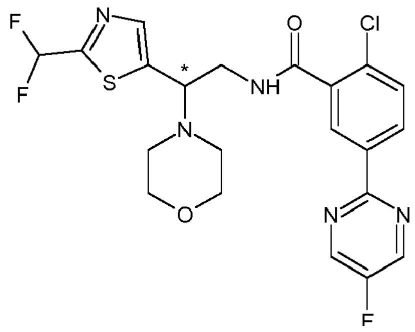 | (+)-2-chloro-N-{2-[2-(difluoromethyl)-1,3-thiazol-5-yl]-2-(morpholin-4-yl)ethyl}-5-(5-fluoropyrimidin-2-yl)benzamide |
| 3 | 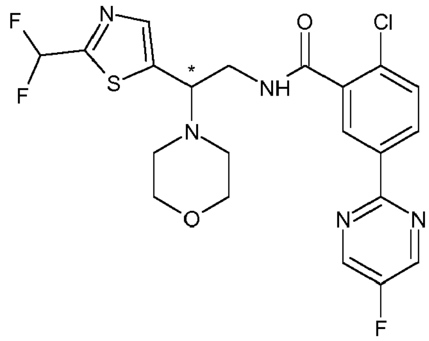 | (-)-2-chloro-N-{2-[2-(difluoromethyl)-1,3-thiazol-5-yl]-2-(morpholin-4-yl)ethyl}-5-(5-fluoropyrimidin-2-yl)benzamide |
| 4 | 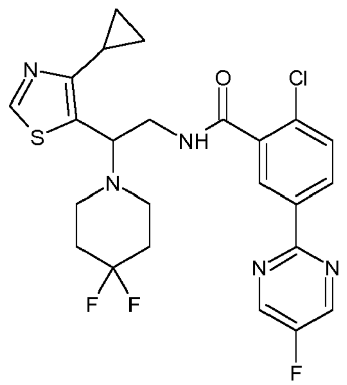 | 2-chloro-N-{2-[4-(cyclopropyl)-1,3-thiazol-5-yl]-2-(4,4-difluoropiperidin-1-yl)ethyl}-5-(5-fluoropyrimidin-2-yl)benzamide |

(continued)

| Compound | Structure | IUPAC Name |
|---|---|---|
| 5 | 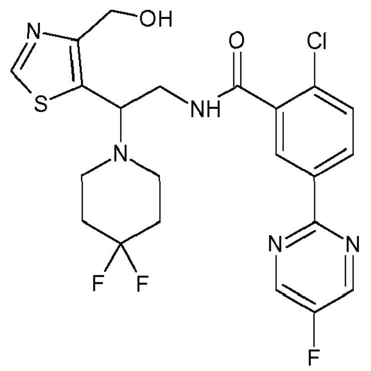 | 2-chloro-N-{2-[4-(hydroxymethyl)-1,3-thiazol-5-yl]-2-(4,4-difluoropiperidin-1-yl)ethyl}-5-(5-fluoropyrimidin-2-yl)benzamide |
| 6 | 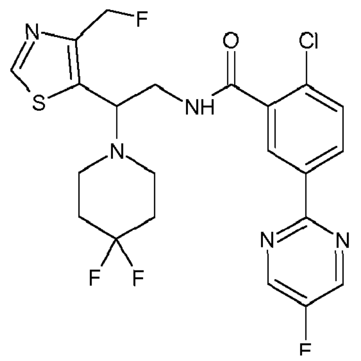 | 2-chloro-N-{2-[4-(fluoromethyl)-1,3-thiazol-5-yl]-2-(4,4-difluoropiperidin-1-yl)ethyl}-5-(5-fluoropyrimidin-2-yl)benzamide |
| 7 | 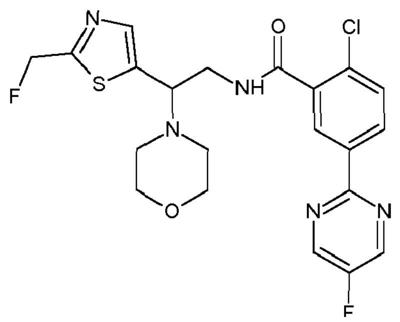 | 2-chloro-N-{2-[2-(fluoromethyl)-1,3-thiazol-5-yl]-2-(morpholin-4-yl)ethyl}-5-(5-fluoropyrimidin-2-yl) benzamide |
| 8 | 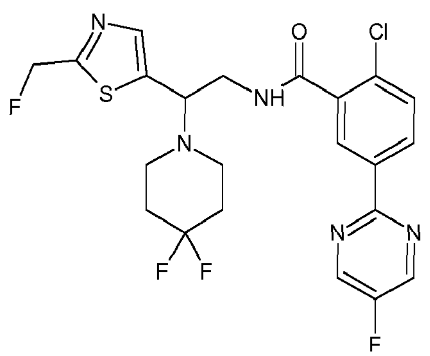 | 2-chloro-N-{2-[2-(fluoromethyl)-1,3-thiazol-5-yl]-2-(4,4-difluoropiperidin-1-yl)ethyl}-5-(5-fluoropyrimidin-2-yl)benzamide |

(continued)

| Compound | Structure | IUPAC Name |
|---|---|---|
| 9 | 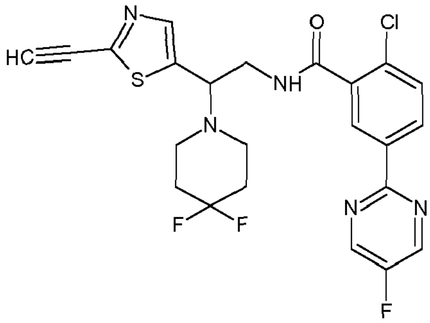 | 2-chloro-N-[2-(4,4-difluoropiperidin-1-yl)-2-(2-ethynyl-1,3-thiazol-5-yl)ethyl]-5-(5-fluoropyrimidin-2-yl)benzamide |

**Analytical part**

**Purification system**

Flash Chromatography (FC)

FC separations were performed on Interchim puriFlash®430, Interchim puriFlash®450 or Interchim puriFlash® 4250-250 equipped with UV detector. Type of silica columns: Interchim puriFlash® SiHP (high performance silica) 50 μm, 4-25 g.

**System purification**

HPLC preparative

HPLC system WATERS Quaternary Gradient Mobile 2535 equipped with WATERS UV/Visible Detector 2489 set to a dual-wavelength UV detection. Two mobile phases were used, mobile phase A: water (MilliQ) 0,05% TFA; mobile phase B: acetonitrile (Chromasolv Sigma-Aldrich) 0,05% TFA, and the run gradient conditions were set specifically for each compound. The purifications were achieved on a LUNA 5 μm C18 150 x 21.2 colum. An injection volume between 100 and 500 μl was used and the flow was 15 ml/ min.

Racemate separation

The two enantiomers 2 and 3 were obtained by resolution of the racemic mixture (Example 1) using a WATERS Quaternary Gradient Mobile 2535 equipped with WATERS UV/Visible Detector 2489 set to a dual-wavelength UV detection at 245 and 260 nm. The chiral resolution was achieved on the Lux Amylose-1 column (150 mm × 21.6 mm, particle size 5 μm) using Hexane-Ethanol 68:32 (v/v) as isocratic mobile phase; The sample was eluted from the column at a flow rate of 15.0 ml/min at room temperature (pressure: ≈ 800 psi). The racemic mixture was dissolved in a mixture Methanol-Ethanol 1:1 and heated, at concentration of 2.5% (w/v) and the injection volume was 200 μL.

*LCMS*

LCMS - procedure 1

The HPLC measurement was performed using a Dionex 3000 module comprising a quaternary pump with degasser, an autosampler, a column oven (set at 29°C), a diode-array detector DAD and a column as specified in the respective methods below. Flow from the column was split to a MS spectrometer. The MS detector (LCQ Fleet Thermo Scientific) was configured with an electrospray ionization source. Mass spectra were acquired by scanning from 50 to 800 in 0.48 second. The capillary needle voltage was 5 kV in positive and negative ionization mode and the source temperature was maintained at 275°C. Nitrogen was used as the nebulizer gas, the flow was 8 l/min. Data acquisition was performed with Thermo Xcalibur Qual Browser. Reversed phase HPLC was carried out on a Kinetex XB-C18 column Phenomenex (1.7 μm, 50 x 2.1 mm) with a flow rate of 0.300 ml/min. Two mobile phases were used, mobile phase A: ammonium formate buffer solution at pH 3.5; mobile phase B: acetonitrile (Chromasolv Sigma-Aldrich), and they were

employed to run a gradient conditions from 15% B to 50% in 15 minutes, 100% B in 0.9 minutes and 5% B in 0.1 minutes and hold these conditions for 4 minutes in order to reequilibrate the column. An injection volume of 1 μl was used.

LCMS - procedure 2

The HPLC measurement was performed using an Agilent 1100 module comprising a quaternary pump with degasser, an autosampler, a column oven (set at 40°), a diode-array detector DAD (wavelength used 215 nm) and a Discovery C18 column Supelco (4.6x 150 mm 5.0 μm) with a flow rate of 1.0 mL/min. Two mobile phases were used, mobile phase A: 0.05% TFA in water (MiliQ) solution; mobile phase B: 0.05% TFA in acetonitrile (HPLC J.T.Baker) solution, and they were employed to run a gradient conditions from 20% B to 90% in 15.0 minutes, 100% B in 9.0 minutes and 20% B in 1. minute and hold these conditions for 4 minutes in ordere to reequilibrate the column. An injection of 5 ul was used. The MS detector (ion trap analyser Esquire 3000 plus Bruker) was configured with an electrospray ionization source. Mass spectra were acquired by scanning from 50 to 1500 Da in 0.2 second. The capillary needle voltage was 4 kV in positive ionization mode and the source temperature was maintained at 365°C. Nitrogen was used as the nebulizer gas the flow was 10.0 l/min. Data acquisition was performed with Data Analysis Bruker Program.

**Table 2:** Retention time ($R_t$) in minutes, $[M+H]^+$peak, LCMS procedure

| **Compound** | **$R_t$ [min]** | **$[M+H]^+$** | **LC-MS procedure** |
|---|---|---|---|
| 1 | 4.8 | 497.9 | 1 |
| 2 | 4.8 | 497.9 | 1 |
| 3 | 4.8 | 497.9 | 1 |
| 4 | 4.5 | 488.0 | 2 |
| 5 | 6.8 | 512.1 | 2 |
| 6 | 9.9 | 513.8 | 2 |
| 7 | 9.3 | 480.8 | 2 |
| 8 | 9.4 | 514.1 | 2 |
| 9 | 7.2 | 506.2 | 2 |

**NMR Characterization**

$^1H$ NMR spectra were recorded on a Bruker Avance III HD 400 MHz (B) or a Varian Mercury NMR 400 MHz (V) spectrometer using $CDCl_3$ or DMSO-d6 as a solvent. Chemical shifts (δ) are reported in parts per million (ppm) relative to residual signal of non-fully deuterated solvents pick for $^1H$ NMR assigned as 7.26 ppm for $CHCl_3$ and 2.50 ppm for DMSO-d6.

| Compound | Spectrometer | $^1H$-NMR |
|---|---|---|
| 1 | V | $^1H$ NMR (400 MHz, Chloroform-d) δ ppm 2.43-2.72 (m, 4 H) 3.74 (br s, 4 H) 3.86 - 3.97 (m, 2 H) 4.19 (br s, 1 H) 6.81 (t, *J* = 54.6 Hz 1 H) 6.85 (br s, 1 H) 7.53 (d, *J*=8.43 Hz, 1 H) 7.76 (br s, 1 H) 8.41 (br d, *J*=8.52 Hz, 1 H) 8.66 (s, 2 H) 8.72 (s, 1 H) |
| 2 | V | $^1H$ NMR (400 MHz, Chloroform-d) δ ppm 2.44-2.55 (m, 2 H) 2.57 - 2.65 (m, 2 H) 3.68 - 3.79 (m, 4 H) 3.90 (br t, *J*=6.09 Hz, 2 H) 4.19 (br t, *J*=7.10 Hz, 1 H) 6.83 (t, *J* = 54.8 Hz 1 H) 6.89 (br s, 1 H) 7.53 (d, *J*=8.43 Hz, 1 H) 7.76 (s, 1 H) 8.41 (dd, *J*=8.43, 1.65 Hz, 1 H) 8.66 (s, 1 H) 8.72 (d, *J*=1.65 Hz, 1 H) |
| 3 | V | $^1H$ NMR (400 MHz, Chloroform-d) δ ppm 2.44-2.55 (m, 2 H) 2.57 - 2.65 (m, 2 H) 3.68 - 3.79 (m, 4 H) 3.90 (br t, *J*=6.09 Hz, 2 H) 4.19 (br t, *J*=7.10 Hz, 1 H) 6.83 (t, *J* = 54.8 Hz 1 H) 6.89 (br s, 1 H) 7.53 (d, *J*=8.43 Hz, 1 H) 7.76 (s, 1 H) 8.41 (dd, *J*=8.43, 1.65 Hz, 1 H) 8.66 (s, 1 H) 8.72 (d, *J*=1.65 Hz, 1 H) |

(continued)

| Compound | Spectrometer | $^1$H-NMR |
|---|---|---|
| 4 | B | $^1$H NMR (400 MHz, DMSO-d6) δ ppm 0.72-1.00 (m, 4 H) 2.08 - 2.18 (m, 1 H) 3.39 (dt, *J*=13.35, 6.42 Hz, 1 H) 3.54 - 3.70 (m, 4 H) 3.89 (dt, *J*=13.22, 6.48 Hz, 1 H) 4.36 (br t, *J*=7.05 Hz, 1 H) 7.64 (d, *J*=7.81 Hz, 1 H) 8.29 - 8.37 (m, 2 H) 8.59 (br t, *J*=5.54 Hz, 1 H) 8.89 (s, 1 H) 9.02 (s, 2 H) |
| 5 | B | $^1$H NMR (400 MHz, DMSO-d6) δ ppm 1.90-2.06 (m, 4 H) 2.54 - 2.69 (m, 4 H) 3.37 - 3.48 (m, 1 H) 3.80 (m, 1 H) 4.57 - 4.64 (br. s, 3 H) 5.18 (t, *J*=5.54 Hz, OH) 7.61 - 7.69 (m, 1 H) 8.30 - 8.37 (m, 2 H) 8.58 (t, *J*=5.67 Hz, NH) 8.96 - 9.05 (m, 3 H) |
| 6 | B | $^1$H NMR (400 MHz, DMSO-d6) δ ppm 1.92-2.09 (m, 4 H) 2.54 - 2.66 (m, 4 H) 3.47 - 3.57 (m, 1 H) 3.74 - 3.87 (m, 1 H) 4.55 (br t, *J*=7.18 Hz, 1 H) 5.42 - 5.53 (q, *J*=14.00, 10.92 Hz, 1 H)) 5.54-5.65 (q, *J*=14.00, 10.92 Hz, 1 H)) 7.65 (d, *J*=8.31 Hz, 1 H) 8.31 (d, *J*=2.01 Hz, 1 H) 8.33 - 8.37 (m, 1 H) 8.62 (t, *J*=5.67 Hz, NH) 9.01 (s, 2 H) 9.14 (s, 1 H) |
| 7 | B | $^1$H NMR (400 MHz, DMSO-d6) δ ppm 2.54 - 2.58 (m, 4 H) 3.55 - 3.66 (br. s, 5 H) 3.85 (dt, *J*=13.28, 6.83 Hz, 1 H) 4.24 (t, *J*=7.30 Hz, 1 H) 5.61 (s, 1 H) 5.72 (s, 1 H) 7.65 (d, *J*=8.31 Hz, 1 H) 7.81 (s, 1 H) 8.31 - 8.32 (m, 1 H) 8.32 - 8.36 (m, 1 H) 8.66 (t, *J*=5.41 Hz, NH) 9.01 (s, 2 H) |
| 8 | B | $^1$H NMR (400 MHz, DMSO-d6 and TFA) δ ppm 2.24 - 2.39 (m, 4 H) 3.27 (br s, 2 H) 3.44 (br s, 2 H) 3.84 - 3.95 (m, 1 H) 4.20 (m, 1 H) 5.22 (m, 1 H) 5.66 (s, 1 H) 5.78 (s, 1 H) 7.57 - 7.64 (m, 1 H) 8.12 (d, J=1.01 Hz, 1 H) 8.23 (d, J=2.27 Hz, 1 H) 8.34 (dd, J=8.56, 2.27 Hz, 1 H) 8.91 (t, J=5.67 Hz, 1 H) 8.96 (d, J=1.26 Hz, 2 H) |
| 9 | B | $^1$H NMR (400 MHz, DMSO-d6) δ ppm 1.92 - 2.07 (m, 4 H) 2.51 - 2.57 (br s, 2 H) 2.61 - 2.75 (m, 2 H) 3.62 (m, 1 H) 3.78 - 3.89 (m, 1 H) 4.40 (t, *J*=7.43 Hz, 1 H) 4.92 (s, 1 H) 7.66 (d, *J*=8.31 Hz, 1 H) 7.87 (s, 1 H) 8.32 - 8.37 (m, 2 H) 8.71 (t, *J*=5.79 Hz, NH) 9.00 (s, 2 H) |

**Pharmacological Examples**

**The Compounds of the invention were found to be active on a human P2X7 channel assay by automated patch-clamp.**

In order to directly monitor the block of P2X7 channel, an electrophysiological assay was developed and implemented on the QPatch16X automated electrophysiology instrument.

HEK-293 cells expressing the P2X7 channels were cultured in modified EMEM.

72 hours before experiment, 5 million cells were seeded onto T225 flasks. Just before the experiment cells were washed twice, detached from the flask with trypsin-EDTA, re-suspended in the suspension solution and placed on the QPatch 16x.

The compounds (20 mM in a 100% DMSO) stored at -20°C were prepared the day of the experiment (a first dilution 1:20 in 100% DMSO to prepare a 1 mM stock solution, then a 1 microM solution in external solution + a serial dilution 1:10).

The standard whole-cell voltage clamp experiments were performed at room temperature. For these experiments the multihole technology was used and the data were sampled at 2 KHz.

The intracellular solution contained (mM) 135 CsF, 10 NaCl, 1 EGTA, 10 HEPES, (pH 7.2 with CsOH) whereas the extracellular contained (mM) 145 NaCl, 4 KCl, 0.5 MgCl2, 1 $CaCl_2$, 10 HEPES, 10 Glc (pH 7.4 with NaOH).

After establishment of the seal and the passage in the whole cell configuration, the cells were held at -80 mV. The P2XR7 current was evoked by applying 100 microM of BzATP alone (4 times) and then in the presence increasing concentrations of the compound under investigation (1, 10, 100 and 1000 nM).

The pre-incubation periods 5 to 8 contain increasing concentrations of the compound of interest (1, 10, 100 and 1000 nM), as illustrated in Figure.

The maximal inward current evoked by BzATP in absence or presence of increasing concentrations of the compounds under investigation was measured and normalized. The potential modulatory effect was measured as % of control and as $IC_{50}$ determined fitting the dose-response curves data with the following equation:

$$Y=100/(1+10^{((LogIC50-X)*HillSlope)})$$

where:

X: log of concentration
Y: normalized response, 100% down to 0%, decreasing as X increases.
Log$IC_{50}$: same log units as X
HillSlope: slope factor or HS, unitless

| Compound | hP2X7 ($IC_{50}$; nM) | ±SEM |
|---|---|---|
| **1** | 47.07 | 5.59 |
| **3** | 26.97 | 4.37 |
| **4** | 103.38 | 9.32 |
| **5** | 64.62 | 6.85 |
| **6** | 33.30 | 4.34 |
| **7** | 38.35 | 7.28 |
| **8** | 20.29 | 3.71 |
| **9** | 39.21 | 10.92 |

**The Compounds of the invention were found to be rat P2X7 inhibitors using a Screen Quest™ Fluo-8 No Wash Calcium Assay Kit.**

$Ca^{2+}$ influx was measured in HEK-293 cells stably transfected with the receptor using Screen Quest™ Fluo-8 No Wash Calcium Assay Kit (AAt Bioquest®). Briefly, once inside the cells, the lipophilic blocking groups of Fluo-8 are cleaved by non-specific cell esterases, resulting in a negatively-charged fluorescent-dye that stays inside cells. Its fluorescence increases upon binding to calcium. When HEK-293/P2X7 cells were stimulated with BzATP, $Ca^{2+}$ entered the cells and the fluorescence of Fluo-8 NW increaseed. The dye absorption spectrum was compatible with excitation at 488 nm by argon laser sources and its emission wavelength was in the range of 515-575 nm.
To routinely test the compounds, HEK-293 cells stably transfected with rat P2X7R were seeded overnight in growth medium at 10000, 15000 or 20000 cells/well in 384-well plate, according to the level of response after thawing. 24 hours later, the medium was removed and the cells were pre-loaded at RT for 1 hour with 20 μL/w of Fluo-8 NW prepared in Tyrode 0.3 mM $Ca^{2+}$ / $Mg^{2+}$ -free.
Compounds of the invention were tested at 8 concentrations (4 replicates for each concentration): 10 - 3.16 - 1 - 0.316 - 0.1 - 0.0316 - 0.01 and 0.00316 μM, in the same plate.
Compounds were tested at FLIPRTETRA according to the following method:

- first injection at FLIPRTETRA of 10 μL of 3x test compound (in Tyrode's buffer 0.3 mM $Ca^{2+}$ / $Mg^{2+}$-free + DMSO 0.5% final concentration)
- 5' incubation
- second injection at FLIPRTETRA of 15 μL of 3x BzATP at ~EC80 (in Tyrode's buffer 0.3 mM $Ca^{2+}$ / $Mg^{2+}$-free + BSA 0.0003% final concentration)
- Fluorescence recording for 3'

Between one plate and the following, tips were extensively washed with water, then with 100% DMSO and finally with water to avoid carry-over inside the tips.
The effect of the test compounds was measured as percent inhibition vs the reference antagonist and $IC_{50}$ values were calculated accordingly.
Compounds of the present invention were found to be unexpectedly 10-12 times more potent than the structurally very similar Example 129 of WO2015118019 as reported in the table 3.

**Table 3**

| **Compounds** | **rP2X7 (IC50; nM)** |
|---|---|
| **1** | 66 |
| **3** | 48 |
| Compound **129** of WO2015118019 | 648 |

**In vitro evaluation of test compounds for metabolic stability using Rat or Mouse liver Microsomes.**

Test System Rat or Mouse Liver Microsomes

Test compound concentration: 1 μM
Time Points: 0, 5, 10, 30 and 60 minutes
Final Protein concentration: 1 mg/mL
Number of Replicates: Two
Potassium Phosphate Buffer pH 7.4 100 mM
End Point: % Remaining of Test compound, Half life, Clint
Bioanalysis by LC-MS/MS

Preparation and Dilution of Test Compound:

10 mM stock solution of test compound were prepared in DMSO and dilute with water: acetonitrile (1:1) to a concentration of 1 mM. Working concentration of 100 μM were prepared by further dilution with water: acetonitrile (1: 1).

Preparation of Potassium Phosphate buffer pH 7.4:

100 mL of Milli Q water will be added to K2HPO4 (1.398 g) and KH2PO4 (0.27 g) to get final pH 7.4.

Assay Procedure:

*Preincubation mixtures*: 2.5 μL Test Cpd. + 75 μL Liver microsomes @ 3.33 mg/mL + 85 μL of 100 mM potassium phosphate buffer (Preincubate for 10 min @ 37°C)
*60 min w/o cofactor:* 32.5 μL of Preincubation mixture + 17.5 μL of 100 mM potassium phosphate buffer (Incubate for 60 min @ 37°C)
*0 min sample:* 16.25 μL of Preincubation mixture + 200 μL of acetonitrile containing internal standard + 8.75 μL of cofactor Incubation mixture 62 μL of cofactor (2.5 mM) + Remaining incubation mixture (Incubated for 60 min@ 37°C).
*Sample preparation:* 25 μL incubation mixture + 200 μL of acetonitrile containing internal standard + Vortex 5 min @ 1200 rpm + Centrifuge 10 min @ 4000 rpm. Dilute supernatant 2 fold with water and injected on LC-MS/MS.
*Bioanalysis:* LC-MS/MS method LC generic gradient conditions will be used and the details will be provided in the report.

Calculation: % remaining of the test substance = [Peak Area at time in min/Peak Area at 0 min]* 100

$K_{el}$: Slope obtained from the plot of Log % remaining vs.time (min)
Half life: $t_{1/2}$ (min) = 0.693/ Kel

$$\text{Intrinsic clearance } (\mu l/min/mg) = ABS\ (K_{el}/\text{Protein Concentration})*1000$$

Examples of the present invention were found unexpectedly to be 1.5-4 times more stable in rat and mouse liver microsomal stability tests as compared to very similar compounds exemplified in WO2015/118019, as reported in the table 4.

**Table 4**

| **Compound** | **Rat Liver Microsomal Clearance ($Cl_i$; μg/min/mg)** | **Mouse Liver Microsomal Clearance ($Cl_i$; μg/min/mg)** |
|---|---|---|
| **4** | 59.41 | 29.0 |
| **6** | - | 47.3 |
| **34** of WO2015/118019 | 156.0 | 121.0 |
| **102** of WO2015/118019 | 121.0 | 69.0 |

## Claims

1. A compound of the following formula (I) or a pharmaceutically acceptable salt thereof:

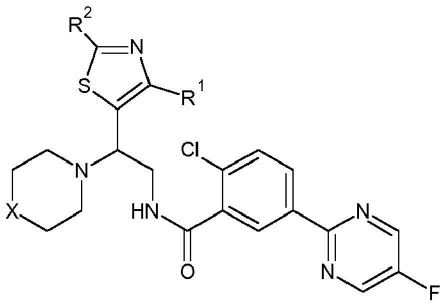

(I)

including any stereochemically isomeric form thereof, wherein

X is O or $CF_2$;
$R^1$ and $R^2$ are independently selected from the group consisting of hydrogen, $CH_2OH$, $CH_2F$, $CHF_2$, cyclopropyl, or -C=CH with the proviso that when one of $R^1$ and $R^2$ is $CH_2OH$, $CH_2F$, $CHF_2$, cyclopropyl or -C=CH, then the other of $R^1$ or $R^2$ group is hydrogen.

2. The compound of formula I according to claim 1, or a pharmaceutically acceptable salt thereof, including any stereochemically isomeric form thereof, selected from the group consisting of:

| | |
|---|---|
| 1 | (±)-2-chloro-N- {2-[2-(difluoromethyl)-1,3-thiazol-5-yl]-2-(morpholin-4-yl)ethyl}-5-(5-fluoropyrimidin-2-yl) benzamide |
| 2 | (+)-2-chloro-N- {2-[2-(difluoromethyl)-1,3-thiazol-5-yl]-2-(morpholin-4-yl)ethyl}-5-(5-fluoropyrimidin-2-yl) benzamide |
| 3 | (-)-2-chloro-N- {2-[2-(difluoromethyl)-1,3-thiazol-5-yl]-2-(morpholin-4-yl)ethyl}-5-(5-fluoropyrimidin-2-yl) benzamide |
| 4 | 2-chloro-N- {2-[4-(cyclopropyl)-1,3-thiazol-5-yl]-2-(4,4-difluoropiperidin-1-yl)ethyl}-5-(5-fluoropyrimidin-2-yl) benzamide |

(continued)

| | |
|---|---|
| 5 | 2-chloro-N- {2-[4-(hydroxymethyl)-1,3-thiazol-5-yl]-2-(4,4-difluoropiperidin-1-yl)ethyl}-5-(5-fluoropyrimidin-2-yl) benzamide |
| 6 | 2-chloro-N- {2-[4-(fluoromethyl)-1,3-thiazol-5-yl]-2-(4,4-difluoropiperidin-1-yl)ethyl}-5-(5-fluoropyrimidin-2-yl) benzamide |
| 7 | 2-chloro-N- {2-[2-(fluoromethyl)-1,3-thiazol-5-yl]-2-(morpholin-4-yl)ethyl}-5-(5-fluoropyrimidin-2-yl)benzamide |
| 8 | 2-chloro-N- {2-[2-(fluoromethyl)-1,3-thiazol-5-yl]-2-(4,4-difluoropiperidin-1-yl)ethyl}-5-(5-fluoropyrimidin-2-yl) benzamide |
| 9 | 2-chloro-N- {2-[2-(ethynyl)-1,3-thiazol-5-yl]-2-(morpholin-4-yl)ethyl}-5-(5-fluoropyrimidin-2-yl)benzamide |

**3.** A process for preparing a compound of formula (I) as defined in claim 1, comprising the step of reacting a compound of formula (II):

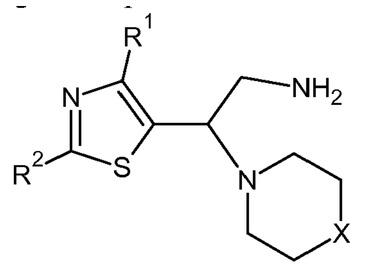

(II)

wherein the meanings of X, $R^1$, and $R^2$ are as defined above, with a compound of formula (III)

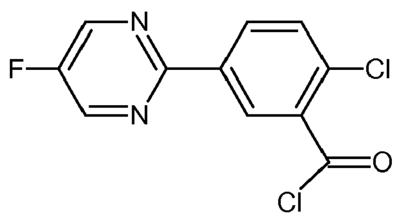

(III)

and optionally converting the obtained compound of formula (I) into a salt thereof, and/or preparing stereochemically isomeric forms thereof.

**4.** A pharmaceutical formulation comprising a compound of formula (I) according to claim 1 or 2, or a pharmaceutically acceptable salt thereof including any stereochemically isomeric form thereof, and a pharmaceutically acceptable diluent and/or carrier.

**5.** A compound of formula (I) according to claim 1 or 2, or a pharmaceutically acceptable salt thereof including any stereochemically isomeric form thereof, for use as a medicament.

**6.** A compound of formula (I) according to claim 1 or 2 for use in prevention and/or treatment of conditions or diseases selected from P2X7 receptor mediated conditions or diseases.

**7.** A compound of formula (I) according to claim 6 for use in prevention and/or treatment of neurodegenerative, cognitive, psychiatric disorders, neuropathic pain, chronic pain, inflammatory processes of the muscoler-skeletal system,

gastrointestinal tract disorders, genitor-urinary tract disorders, ophthalmic diseases.

**Figure**

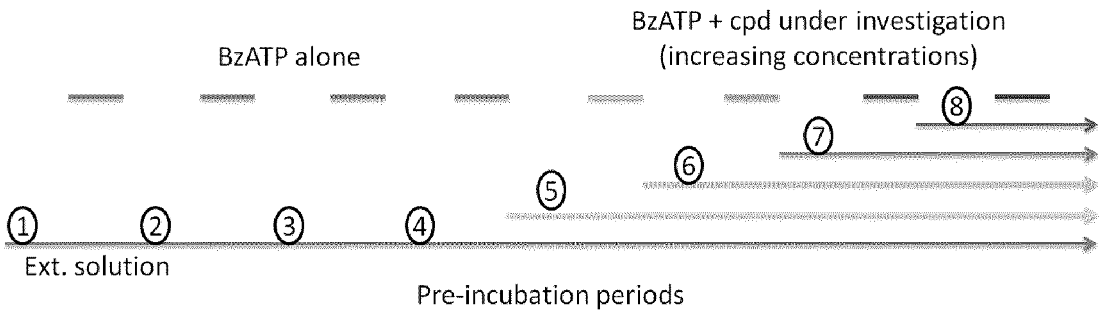
BzATP + cpd under investigation
(increasing concentrations)
BzATP alone
8
7
6
5
1
2
3
4
Ext. solution
Pre-incubation periods

Europäisches Patentamt
European Patent Office
Office européen des brevets

# EUROPEAN SEARCH REPORT

Application Number
EP 16 18 6658

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X,D | WO 2015/118019 A1 (AXXAM S P A [IT]) 13 August 2015 (2015-08-13) * the whole document; in particular, claims 1 and 8-12, page 72, compound 20, page 79, compound 87, page 81, compound 102, page 82, compound 112, page 83, compound 129, and, in particular, page 82, compound 119 * ----- | 1-7 | INV. C07D417/12 C07D417/14 A61K31/506 A61P1/00 A61P13/00 A61P21/00 A61P25/28 A61P27/02 A61P29/00 |
| | | | TECHNICAL FIELDS SEARCHED (IPC) |
| | | | C07D |

The present search report has been drawn up for all claims

3

| Place of search | Date of completion of the search | Examiner |
|---|---|---|
| Munich | 6 October 2016 | Fink, Dieter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 16 18 6658

This annex lists the patent family members relating to the patent documents cited in the above-mentioned European search report. The members are as contained in the European Patent Office EDP file on
The European Patent Office is in no way liable for these particulars which are merely given for the purpose of information.

06-10-2016

| Patent document cited in search report | Publication date | Patent family member(s) | Publication date |
| --- | --- | --- | --- |
| WO 2015118019 A1 | 13-08-2015 | CA 2938703 A1 | 13-08-2015 |
| | | EP 2905282 A1 | 12-08-2015 |
| | | WO 2015118019 A1 | 13-08-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004099146 A
- WO 2009108551 A
- WO 2009132000 A
- WO 2015118019 A


### Non-patent literature cited in the description


- **J.I. DIAZ-HERNANDEZ et al.** *Neurobiol. Aging,* 2012, 1816-1828
- **THEODORA W. GREENE ; PETER G.M. WUTS.** Protective groups in organic synthesis. John Wiley & Sons, Inc, 1991